# EUROPEAN PATENT SPECIFICATION

(11) **EP 1 716 887 B1**
(45) Date of publication and mention of the grant of the patent: **19.03.2008**
(21) Application number: 05008993.7
(22) Date of filing: 25.04.2005
(51) Int. Cl.: A61P 21/00, A61K 38/25, A61K 38/27, A61K 38/30

(54) **Treatment of inclusion body myositis**
Behandlung von Einschlusskörper-Myositis
Traitement de la Myosite corps d'inclusion

(43) Date of publication of application: 02.11.2006
(73) Proprietor: Sahltech I Göteborg AB, 413 45 Göteborg (SE)
(72) Inventor: Olbe, Lars, 412 53 Göteborg (SE); Rosén, Thord, 436 54 Hovas (SE)
(74) Representative: Becker Kurig Straus

(56) References cited:
- WO-A-20/05034858
- US-A1- 2004 248 766
- GASPARINI L ET AL: "Potential roles of insulin and IGF-1 in Alzheimer's disease" TRENDS IN NEUROSCIENCE, ELSEVIER, AMSTERDAM, NL, vol. 26, no. 8, August 2003 (2003-08), pages 404-406, XP004443472 ISSN: 0166-2236
- BROCCOLINI ALDOBRANDO ET AL: "Insulin-like growth factor I in inclusion-body myositis and human muscle cultures" JOURNAL OF NEUROPATHOLOGY & EXPERIMENTAL NEUROLOGY, vol. 63, no. 6, June 2004 (2004-06), pages 650-659, XP009052734 ISSN: 0022-3069
- LYNCH G S ET AL: "IGF-I treatment improves the functional properties of fast- and slow-twitch skeletal muscles from dystrophic mice." NEUROMUSCULAR DISORDERS : NMD. APR 2001, vol. 11, no. 3, April 2001 (2001-04), pages 260-268, XP002341784 ISSN: 0960-8966
- KOKONTIS LISA ET AL: "Current treatment of neuromuscular diseases" ARCHIVES OF NEUROLOGY, vol. 57, no. 7, July 2000 (2000-07), pages 939-943, XP009052737 ISSN: 0003-9942

## Description

The present invention relates to the field of myopathy and particularly to inclusion body myositis (IBM). In particular the present invention envisages the use of a composition, comprising as an active ingredient either growth hormone, a secretagogue thereof or a mixture thereof, for the manufacture of a medicament for the treatment and/or prevention of IBM and/or suppression of symptoms associated therewith.

Myopathy, a generic term designating a neuromuscular disease, is generally characterized by a dysfunction of muscle fibers and results in muscular weakness up to a complete muscle degradation. Muscle cramps, stiffness and spasms are examples of symptoms often associated with this disease condition.

Myopathy may be subdivided into several forms, preliminary depending on the site the disease shows up, the symptoms associated therewith and on the physiological pathways involved. Examples for the different types of myopathy comprise dysphagia, fibrotic myopathy, different kinds of mitochondrial myopathy, muscular dystrophy, myositis and myotonic myopathy.

In the art, there is no unique treatment for myopathy as such since there seem to be a variety of different causes why said disease is elicited. Presently applied therapies commonly involve treatment of the symptoms only to treatment of the specific causes. Drug therapy, physical therapy or physiotherapy, bracing for support, surgery, and even acupuncture are examples of current treatments for a number of myopathies.

Examples of drugs used for the treatment oft myopathy may include non-steroidal antiinflammatories, analgesics, sedatives and antidepressants including selective serotonin reuptake inhibitors. Opioids are occasionally used for the treatment of myopathy related illnesses, said compounds are however generally not recommended because of the high risk of abuse.

Inclusion body myositis (IBM) is a specific form of myopathy. The term "inclusion body myositis" was originally used by Yunis and Samaha in 1971 for a particular case of myopathy that phenotypically suggested the presence of chronic polymyositis, but showed cytoplasmic vacuoles and inclusions on muscle biopsy. IBM seems to be a chronic inflammatory myopathy with a doubled to tripled predominance in male and is apparently both an inflammatory and a degenerative muscle disease. The two known forms of IBM comprise the sporadic inclusion body myositis (s-IBM) and inherited inclusion body myopathies (i-IBM).

IBM is the most frequent acquired neuromuscular disorder occurring in patients with an age over 50 years, the mean age of the disease debut being about 60 years. It is characterized by an insidious, steadily progressive course of asymmetric muscle atrophy and muscle weakness of both proximal and distal involvement with an approximate loss of muscle power of 12% per year. Ten years after the diagnosis about half of the patients are confined to a wheel-chair and after 15 years the majority of the patients require a major assistance for their daily lives, whereas the incidence of morbidity is not assessable. The cause for said slowly progressive illness is up to now unclear.

Although light microscopy of biopsy specimen typically demonstrates signs of inflammatory myositis, the findings in the muscle tissues are characterized by 1) cavities in the muscle fibres, the so called "rimmed vacuoles"; 2) tubular filaments of about 15 mm in diameter (mainly composed of hyperphosphorylated tau- and beta-amyloid inclusions); and 3) the abnormal accumulation of proteins commonly observed in Alzheimer disease. The pathogenic cause to IBM remains undefined, although an autoimmune aetiology has been discussed.

Up to now there is no standard course to prevent or retard the progressive, negative outcome of IBM. It is reported that the disease is unresponsive to corticosteroids and immunosuppressive drugs. Some evidence suggests that intravenous immunoglobulin may have a slight, but short-lasting, beneficial effect in a small number of cases. Physical therapy may be helpful in maintaining mobility. Other therapies have merely proven symptomatic and supportive effects. Accordingly, an effective, physiological way to treat IBM is highly desirable.

Thus, an object of the present invention resides providing means for effectively treating and/or preventing IBM. Another objective resides in providing a treatment regimen, which has less or no detrimental side effects and is nevertheless highly effective.

This objective has been achieved by the use of a composition, which comprises as an active ingredient at least one constituent selected from among growth hormone and/or a growth hormone secretagogue. It has surprisingly been found that said compounds either alone or in a combination exhibit the capacity to alleviate or even remove symptoms associated with IBM.

According to a first embodiment of the present invention, the use of a composition for the manufacture of a medicament for the prevention and/or treatment of MBI is envisaged. Said composition comprises as an active ingredient growth hormone and/or a growth hormone secretagogue.

Growth hormone (GH), also known as somatotropin, is a protein hormone of about 191 amino acids (22 kD) that is synthesized and secreted by the somatotroph cells in the anterior lobe of the pituitary. Hypothalamic peptides regulate its synthesis, wherein growth hormone releasing hormone (GHRH, also designated GRF) stimulates and somatostatin inhibits its release. Growth hormone is a major participant in control of several complex physiologic processes, including growth and metabolism. In addition, growth hormone is known to have the following basic effects on the metabolic processes of the body: an increased rate of protein synthesis in the cells; a decreased rate of carbohydrate utilization in the cells; and an increased mobilization of free fatty acids and their conversion to energy.

The GH secretion has usually a diurnal rhythm, and is stimulated during sleep with a peak occurring in the early morning before awakening, and the lowest values during the day. The pulsatile secretion pattern gives a great variation of serum GH levels, from zero to peaks of 30 ng/ml. The circulating halftime for GH is about 20 minutes, and GH is degraded mainly in the liver and the kidney. Apart from sleep, GH secretion is stimulated by hypoglycaemia, certain amino acids such as arginine and leucine, exercise and stress. The secretion is decreased by hyperglycaemia. The GH production is highest during puberty, and is then continuously decreased with age. GH stimulates growth and has metabolic actions. In general, it has anabolic actions with stimulated protein synthesis. Furthermore, it shifts metabolism to use lipids for energy, thereby conserving carbohydrates and protein. GH administration causes increased skeletal and visceral growth; without GH children show growth failure.

Anabolic action of GH includes increased cellular amino acid uptake with incorporation into protein. It causes a positive nitrogen balance with nitrogen retention and a decreased urea production. GH is lipolytic via activation of hormone-sensitive lipase and secondary mobilisation of neutral fats from adipose tissue, giving uptake and oxidation of fatty acid in skeletal muscle and liver. GH has a mild hyperglycaemic effect via an antagonistic effect on insulin action at the post-receptor level in skeletal muscle and adipose tissue and by increasing the hepatic output of glucose.

The GH to be used in the present invention may be naturally derived GH, such as extracted from pituitary glands. However, since the extraction and/or purification is costly and carries the risk that a disease associated with the source of the pituitary gland may be transmitted to the recipient of the growth hormone, recombinant growth hormone is preferably utilized.

Alternatively, or in addition to GH, compounds that result in the or an increased production/secretion of GH may be used. Such compounds are generically referred to as Growth Hormone Secretagogues and comprise e.g. MK-0677, L-162752 and L-163022 (Merck); NN703 and ipamorelin (Novo Nordisk); hexarelin (Pharmacia); GPA-748 (KP102, GHRP-2) (American Home Products); and LY444711 (EliLilly). The following compounds have been reported to be capable to stimulate GH release via the GHRH/GRF receptor (including GHRH/GRF derivatives, analogues and mimetics) are for example Geref (Ares/Serono); GHRH (1-44) (BioNebraska); Somatorelin (GRF 1-44) (Fujisawa/ICN) and ThGRF (Theratechnologies). A preferred hrowth hormone secretagogue is genotropin (Pfizer).

Representative growth hormone secretagogues are disclosed in the following international documents: WO 98/46569, WO 98/51687, WO 98/58947, WO 98/58949, WO 98/58950, US-P-6,127,341, WO 99/08697, WO 99/09991, WO 95/13069, WO 95/14666, and WO 94/19367.

WO 94/13696, WO 94/11012, WO 95/11029, WO 95/17422, WO 95/17423, US-P-5,767,085, WO 95/34311, US-P-5,777,112, WO 96/02530, US-P-5,783,582, WO 96/22996, WO 96/22997, US-P-6,013,658, WO 96/24580, WO 96/24587, US-P-5,559,128, WO 96/32943, WO 96/33189, WO 96/15148, US-P-5,798,337, WO 96/38471, US-P-5,936,089, WO 96/35713, WO 97/00894, WO 97/07117, WO 97/06803, US-P-5,773,441, WO 97/11697, WO 97/15573, US-P-5,723,616, WO 97/22367, US-P-5,830,433, WO 97/23508, US-P-5,977,178, WO 97/22620, WO 97/22004, US-P-6,242,199, WO 97/21730, US-P-6,531,314, WO 97/24369, US-P-6,107,306, US-P-5,663,171, WO 97/34604, US-P-5,880,125, WO 97/36873, US-P-5,804,578, WO 97/40071, US-P-5,990,084, WO 97/40023, US-P-5,919,777, WO 97/41878, WO97/41879, WO 97/46252, WO 97/44042, US-P-6,071,926, WO 97/38709, WO 98/03473, US-P-5,922,770, WO 97/43278, US-P-6,127,343, US-P-5,721,251, US-P-5,721,250, WO 98/10653, WO 00/01726, US-P-5,830,433 and EP 0995748.

A growth hormone secretagogue is an exogenously administered compound or agent that directly or indirectly stimulates or increases the endogenous release of growth hormone, growth hormone-releasing hormone or somatostatin in an animal, in particular, a human. Growth hormone secretagogues may be peptidyl or non-peptidyl in nature, however, the use of an orally active growth hormone secretagogue is preferred. In addition, it is preferred that the growth hormone secretagogue induces or amplifies a pulsatile release of endogenous growth hormone. Examples of GH or GH secretagogue administration are disclosed in the US application 2005065180, wherein the treatment of osteoporosis like diseases is envisaged.

Other possibilities to stimulate the release of the growth hormone are disclosed for example in Recent Progress in Hormone Research, vol. 52, pp. 215-245 (1997); and Front Horm. Res. Basel, Karger, vol. 24, pp. 152-175 (1999). For example, chemicals such as arginine, L- 3,4-dihydroxyphenylalanine (L-DOPA), glucagon, vasopressin, and insulin induced hypoglycemia, as well as activities such as sleep and exercise, indirectly cause growth hormone to be released from the pituitary by acting in some fashion on the hypothalamus perhaps either to decrease somatostatin secretion or to increase secretion of growth hormone releasing factor (GRF) or ghrelin (Nature, vol. 402, pp. 656-660 (9 December 1999)).

According to an embodiment, said growth hormone secretagogue is selected from the group consisting of genotropin, ghrelin and hexarelin.

According to an embodiment of the present invention, said composition comprises further insulin-like growth factor-1 (IGF-1). It is already known in the art that in cases in which increased levels of growth hormone are desired, exogenous growth hormone was provided or GRF, IGF-I or a peptidyl compound, which stimulated growth hormone production and/or release. In the course of the extensive studies also surprisingly a synergistic effect for the treatment and/or prevention of IBM has been observed at concomitant administration of IGF-1.

Without wishing to be bound by any theory, it is presently assumed that the use of a composition comprising somatotropin and/or a growth hormone secretagogue and IGF-1 has a positive effect on both an increased bioavailability of GH and/or GH secretagogue and additionally on the abnormal production of beta-amyloid, the premature muscle aging and the increased oxidative stress. All three factors represent the major possible causes of the up to now incurable IBM.

IGF-I, which is predominantly produced in the liver via GH from the pituitary, is a pleiotropic growth factor with both endocrine and autocrine/paracrine functions, and has a role for regeneration and growth in the muscle. Furthermore, IGF-I seems to have a protective role against beta-amyloid toxicity, and to reduce the harmful effect of stress and finally increase the survival function of the cell.

IGF-I may be produced or obtained by any suitable, usually recombinant, means known to the skilled person. For example, mRNA encoding an isoform may be amplified using PCR primers and the amplified product inserted into a suitable expression vector. The vector may be any suitable recombinant vector known in the art for recombinant proteins. The vector contains control signals for the expression of the IGF-I protein. The promoter used is compatible with a suitable host cell, for example a bacterial, yeast, insect or mammalian cell.

According to another embodiment, said composition is in the form of a nasal, inhalable, topical, parenteral or transdermal formulation. Preferably, if growth hormone is used, an transdermal administration via injection is chosen, whereas the use of a growth hormone secretagogue permits the application of other formulations.

Due to the peptidyl nature of said compound administration via injection is advisable.

Formulations suitable for respiratory, nasal, intrapulmonary, and inhalation administration are preferred, as are topical, oral and parenteral formulations. All methods of preparation may include the step of bringing the active compound(s) into association with a carrier, which constitutes of one or more accessory ingredients. In general, the formulations are prepared by uniformly and intimately bringing the active compound into association with a liquid carrier, a finely divided solid carrier, or both, and then, if necessary, shaping the product into desired formulations.

Suitable compositions for oral administration may be presented in discrete units, such as capsules, cachets, lozenges, or tablets, each containing a predetermined amount of the active compound; as a powder or granules; as a solution or a suspension in an aqueous or non-aqueous liquid; or as an oil-in-water or water-in-oil emulsion. Such compositions may be prepared by any suitable method of pharmacy, which includes the step of bringing into association the active compound and a suitable carrier. In general, the compositions are prepared by uniformly and intimately admixing the active compound with a liquid or finely divided solid carrier, or both, and then, if necessary, shaping the resulting mixture. For example, a tablet may be prepared by compressing or molding a power or granules containing the active compound, optionally with one or more accessory ingredients. Compressed tablets may be prepared by compressing in a suitable machine, the compound in a free-flowing form, such as a powder or granules optionally mixed with a binder, lubricant, inert diluent, and/or surface active/dispensing agent(s). Molded tablets may be prepared by molding, in a suitable machine, the powdered compound moistened with an inert liquid binder. A syrup may be made by adding the active compound to a concentrated aqueous solution of a sugar, for example sucrose to which may also be added any accessory ingredient(s). Such accessory ingredient(s) may include flavourings, suitable preservatives, an agent to retard crystallization of the sugar, and an agent to increase the solubility of any other ingredient, such as a polyhydric alcohol, for example glycerol or sorbitol. Compositions for oral administration may optionally include enteric coatings known in the art to prevent degradation of the compositions in the stomach and provide release of the drug in the small intestine. Compositions suitable for buccal or sub-lingual administration include lozenges comprising the active compound in a flavoured base, usually sucrose and acacia or tragacanth and pastilles comprising the compound in an inert base such as gelation and glycerin or sucrose and acacia.

Compositions suitable for parenteral administration comprise sterile aqueous and non-aqueous injection solutions of the active compound, which preparations are preferably isotonic with the blood of the intended recipient.

These preparations may contain anti-oxidants, buffers, bacteriostats and solutes, which render the compositions isotonic with the blood of the intended recipient. Aqueous and non-aqueous sterile suspensions may include suspending agents and thickening agents. The compositions may be presented in unit-dose or multi-dose containers, for example sealed ampoules and vials, and may be stored in a freeze-dried or a lyophilized condition requiring only the addition of the sterile liquid carrier, for example, saline or water-for-injection immediately prior to use. Extemporaneous injection solutions and suspensions may be prepared from sterile powders, granules and tablets of the kind previously described.

Nasal and instillable formulations comprise purified aqueous solutions of the active compound with preservative agents and isotonic agents. Such formulations are preferably adjusted to a pH and isotonic state compatible with the nasal mucous membranes.

Ophthalmic formulations are prepared by a similar method to the nasal spray, except that the pH and isotonic factors are preferably adjusted to match that of the eye. Otical formulations are generally prepared in viscous carriers, such as oils and the like, as is known in the art, so that they may be easily administered into the ear without spilling.

Compositions suitable for topical application to the skin preferably take the form of an ointment, cream, lotion, paste, gel, spray, aerosol, or oil. Carriers, which may be used include vaseline, lanolin, polyethylene glycols, alcohols, transdermal enhancers, and combinations of two or more thereof. Compositions suitable for transdermal administration may be presented as discrete patches adapted to remain in intimate contact with the epidermis of the recipient for a prolonged period of time. Compositions suitable for transdermal administration may also be delivered by iontophoresis and typically take the form of an optionally buffered aqueous solution of the active compound. Topical formulations comprise the active compound dissolved or suspended in one or more media such as mineral oil, petroleum, polyhydroxy alcohols or other bases used for topical pharmaceutical formulations. Cosmetic formulations may be in the form of solid or liquid preparations, for spreading on a subject's skin, including skin base, pancake, suntan, self-tanning and sun blocking lotions and oils.

These formulations may additionally contain other cosmetic ingredients as are known in the art. Examples of these formulations are lotions, creams, oils, and other ointments, e. g. lotions containing sunscreens and other protective ingredients, facial make-up and cleansing formulations, shampoos, hair and skin conditioners, and many more known in the art and commercially available. The addition of other accessory ingredients, vide infra, may be desirable, for example, accessory ingredient (s) selected from diluents, buffers, flavouring, colouring and aromatizing agents, binders, disintegrants, surface active agents, thickeners, lubricants, emulsifiers, surfactants, emollients, preservatives (including anti-oxidants), and the like. Other ingredients may also be utilized as is known in the art.

The active compounds disclosed herein may be administered into the respiratory system either by inhalation, respiration, nasal administration or intrapulmonary instillation (into the lungs) of a subject by any suitable means, and are preferably administered by generating an aerosol or spray comprised of powdered or liquid nasal, intrapulmonary, respirable or inhalable particles. The respirable or inhalable particles comprising the active compound are inhaled by the subject, i. e, by inhalation or by nasal administration or by instillation into the respiratory tract or the lung itself.

The formulation may comprise respirable or inhalable liquid or solid particles of the active compound that, in accordance with the present invention, include respirable or inhalable particles of a size sufficiently small to pass through the mouth and larynx upon inhalation and continue into the bronchi and alveoli of the lungs. In general, particles ranging from about 0.05 up to about10 microns in size. More particularly, about 0.5 to less than about 5 microns in size, are respirable or inhalable. Particles of non-respirable size, which are included in an aerosol or spray tend to deposit in the throat and be swallowed. The quantity of non-respirable particles in the aerosol is, thus, preferably minimized. For nasal administration or intrapulmonary instillation, a particle size in the range of about 10 up to about 500 µm is preferred to ensure retention in the nasal cavity or for instillation and direct deposition into the lung. Liquid formulations may be squirted into the respiratory tract (nose) and the lung, particularly when administered to newborns and infants.

Liquid pharmaceutical compositions of active compound for producing an aerosol may be prepared by combining the active compound with a stable vehicle, such as sterile pyrogen free water. Solid particulate compositions containing respirable dry particles of micronized active compound may be prepared by grinding dry active compound with a mortar and pestle, and then passing the micronized composition through a 400mesh screen to break up or separate out large agglomerates. A solid particulate composition comprised of the active compound may optionally contain a dispersant that serves to facilitate the formation of an aerosol. A suitable dispersant is lactose, which may be blended with the active compound in any suitable ratio, e. g. a 1 to 1 ratio by weight. Aerosols of liquid particles comprising the active compound may be produced by any suitable means, such as with a nebulizer. See, e. g. US Patent No. 4,501, 729. Nebulizers are commercially available devices which transform solutions or suspensions of the active ingredient into a therapeutic aerosol mist either by means of acceleration of a compressed gas, typically air or oxygen, through a narrow venturi orifice or by means of ultrasonic agitation. Suitable compositions for use in nebulizer consist of the active ingredient in liquid carrier, the active ingredient comprising up to 40% w/w composition, but preferably less than 20%w/w carrier being typically water or a dilute aqueous alcoholic solution, preferably made isotonic with body fluids by the addition of, for example sodium chloride. Optional additives include preservatives if the composition is not prepared sterile, for example, methylhydroxybenzoate, anti-oxidants, flavouring agents, volatile oils, buffering agents and surfactants. Aerosols of solid particles comprising the active compound may likewise be produced with any sold particulate medicament aerosol generator. Aerosol generators for administering solid particulate medicaments to a subject product particles which are respirable, as explained above, and generate a volume of aerosol containing a predetermined metered dose of a medicament at a rate suitable for human administration. Examples of such aerosol generators include metered dose inhalers and insufflators.

According to another embodiment, said growth hormone or growth hormone secretagogue is contained in an amount of about 0.1 to 2.0 mg per single (daily) dose. Preferably, the growth hormone or growth hormone secretagogue is contained in an amount of about 0.2 to 1.0 mg and more preferably in an amount of about 0.4 to 0.6 mg per single (daily) dose.

According to still another embodiment said IGF-1 is contained in an amount of about 0.05 to 1.0 mg per single (daily) dose. Preferably, the IGF-1 is contained in an amount of about 0.05 to 0.6 mg and more preferably in an amount of about 0.1 to 0.4 mg per single (daily) dose.

In general, the active agents of this invention are provided within broad amounts in the composition depending on e.g. absorption, inactivation, excretion rates, the dosage schedule and amount administered as well as other factors known to those of skilled in the art. The dosage of the active compounds, however, may vary depending on age, weight, and condition of the subject. Treatment may be initiated with a small dosage, e. g. less than the optimal dose, of the first active agent of the invention. This may be similarly done with a second active agent, until a desirable level is attained. The dose may be increased until a desired and/or optimal effect under the circumstances is reached. In general, the active agent is preferably administered at a concentration that will afford effective results without causing any unduly harmful or deleterious side effects, and may be administered either as a single unit dose, or if desired in convenient subunits administered at suitable times throughout the day. The active ingredient(s) may be also administered in form of a pharmaceutically acceptable salt. Pharmaceutically acceptable salts should be pharmacologically and pharmaceutically acceptable, and may be prepared as alkaline metal or alkaline earth salts, such as sodium, potassium or calcium salts. Organic salts and esters are also suitable for use with this invention. The active compounds are preferably administered to the subject as a pharmaceutical composition, which includes systemic and topical formulations. Among these, preferred are formulations suitable for inhalation, or for respirable, buccal, oral, rectal, vaginal, nasal, intrapulmonary, ophthalmic, optical, intracavitary, intratraccheal, intraorgan, topical (including buccal, sublingual, dermal and intraocular), parenteral (including subcutaneous, intradermal, intramuscular, intravenous and intraarticular) and transdermal administration, slow release, implantable, and enteric coated, among others. The compositions may conveniently be presented in single or multiple unit dosage forms as well as in bulk, and may be prepared by any of the methods, which are well known in the art of pharmacy. The actual preparation and compounding of these different formulations is well known in the art. The active compounds may be administered once or several times a day. The composition of the invention may also be provided in the form of a kit, whether already formulated or where the active agents are separately provided along with other ingredients, and instructions for its formulation and administration regime. The kit may also contain other agents, such as those described in this patent and, for example, when for parenteral administration, they may be provided with a carrier in a separate container, where the carrier may be sterile.

The following examples illustrate the invention without limiting it thereto.

### Examples

The patient is a 71-year old, an ex-smoker and is on continuous anti-coagulation therapy, due to multiple deep vein thrombosis in turn secondary to APC-resistance. In 1992 muscle weakness in the legs was noticed accompanied by difficulties to raise from a chair to a standing position and to walk longer distances. In 1996 years ago, also weakness in the arms was noticed and subsequently progressive muscle weakness together with some breathing difficulties and numbness of the extremities occurred. In 2001 the diagnosis of IBM was made, and the patient was given immunoglobulin. Immunoglobulin was withdrawn due to no positive effect. Half a year ago, an ACB-operation was performed, and the patient was given a mechanical aortic valve, with a prompt regress of the cardiac failure. In April 2002 GH-treatment was started trying to reduce the progress of the IBM-disease. After some months treatment with genotropin (Pfizer) 0.4 mg sc (subcutaneous) daily, the patient subjectively noticed increased muscle strength in both his arms and legs, together with an increase in walking distances and in general well-being. After one year of GH-treatment with the dose 0.4-0.5 mg sc daily an objective improvement in spirometry and muscle power was noticed. No side-effects were noted, and the IGF-I concentration has been within the normal range. After two years treatment the patient had a silent myocardial ischemia after a varix operation, with a post-operative cardiac failure, which was successfully treated with diuretics and continuous GH-treatment. Some month later the patient was also successfully recovered from a serious sepsis, which required some weeks of ICU-treatment. The patient is extremely satisfied with the GH-treatment, that has stopped the down-hill course of the disease and even caused an improvement of the muscle power.

## Claims

1. Use of a composition comprising growth hormone and/or a growth hormone secretagogue for the preparation of a medicament for the treatment and/or prevention of (inclusion body myositis) IBM.

2. The use according to claim 1, wherein that said composition further comprises IGF-1.

3. The use according to claim 1 or 2, wherein said composition is in the form of a nasal, inhalable, topical, parenteral or transdermal formulation.

4. The use according to claim 1, **characterized in that** said growth hormone secretagogue is selected from the group consisting of genotropin, ghrelin and hexarelin.

5. The use according to claim 1, wherein said growth hormone or growth hormone secretagogue is contained in an amount of 0.1 to 2.0 mg.

6. The use according to claim 2, wherein said IGF-1 is contained in an amount of 0.05 to 1.0 mg.

## Patentansprüche

1. Verwendung einer Zusammensetzung, welche Wachstumshormon und/oder eine Wachstumshormon-Secretagogue umfasst, zur Herstellung eines Medikaments für die Behandlung und/oder Verbeugung von Einschlusskörpermyositis IBM.

2. Verwendung nach Anspruch 1, wobei die Zusammensetzung weiterhin IGF-1 umfasst.

3. Verwendung nach Anspruch 1 oder 2, wobei die Zusammensetzung in der Form einer nasalen, inhalierbaren, topischen, parenteralen oder transdermalen Formulierung vorliegt.

4. Verwendung nach Anspruch 1, **dadurch gekennzeichnet, dass** die Wachstumshormon-Secretagogue ausgewählt ist aus der Gruppe bestehend aus Genotropin, Ghrelin und Hexarelin.

5. Verwendung nach Anspruch 1, wobei das Wachstumshormon oder die Wachstumshormon-Secretagogue in einer Menge von 0,1 bis 2,0 mg enthalten ist.

6. Verwendung nach Anspruch 2, wobei das IGF-1 in einer Menge von 0,05 bis 1,0 mg enthalten ist.

## Revendications

1. Utilisation d'une composition comprenant l'hormone de croissance et/ou un sécrétagogue de l'hormone de croissance pour la préparation d'un médicament pour le traitement et/ou la prévention de la myosite corps d'inclusion IBM.

2. L'utilisation selon la revendication 1, dans laquelle ladite composition comprend en outre l'IGF-1.

3. L'utilisation selon la revendication 1 ou 2, dans laquelle ladite composition est sous forme d'une formulation nasale, inhalable, topique, parentérale ou transdermique.

4. L'utilisation selon la revendication 1, dans laquelle ledit sécrétagogue d'hormone de croissance est sélectionné à partir du groupe consistant en génotropine, ghreline et hexareline.

5. L'utilisation selon la revendication 1, dans laquelle ladite hormone de croissance ou sécrétagogue de l'hormone de croissance est contenu en une quantité de 0.1 à 2.0 mg.

6. L'utilisation selon la revendication 2, dans laquelle ledit IGF-1 est contenu en une quantité de 0.05 à 1.0 mg
